# FASCICULE DE BREVET EUROPEEN

(11) **EP 0 442 992 B1**
(45) Date de publication et mention de la délivrance du brevet: **07.06.1995**
(21) Numéro de dépôt: 90912805.0
(22) Date de dépôt: 05.09.1990
(51) Int. Cl.: A61N 1/40

(54) **APPAREIL D'ELECTROMAGNETOTHERAPIE**
ELEKTROMAGNETOTHERAPIEGERÄT
ELECTROMAGNETIC THERAPY DEVICE

(30) Priorité: 12.09.1989 CH 3330/89
(43) Date de publication de la demande: 28.08.1991
(73) Titulaire: ANNONI DI GUSSOLA, Guido P., F-01210 Ferney-Voltaire (FR)
(72) Inventeur: ANNONI DI GUSSOLA, Guido P., F-01210 Ferney-Voltaire (FR)
(74) Mandataire: KIRKER & Cie S.A.
(86) Numéro de dépôt international: EP9001543
(87) Numéro de publication internationale: WO9104070

(56) Documents cités:
- EP-A- 0 076 074
- WO-A-88/09685
- BE-A- 677 767
- DE-A- 3 244 582
- FR-A- 2 291 773
- FR-A- 2 386 912

## Description

La présente invention concerne un appareil d'électromagnétothérapie, comprenant un générateur d'ondes électromagnétiques susceptible de générer une onde mégahertzienne, des premiers moyens pour produire l'émission de trains de cette onde mégahertzienne d'une première durée prédéterminée intercalés entre des intervalles d'interruption d'une seconde durée prédéterminée et des seconds moyens pour inhiber l'émission desdits trains pour former des pauses d'une troisième durée prédéterminée réglable, le générateur comportant au moins un dispositif émetteur.

En 1900, le médecin russe Danilewski a étudié l'action des ondes hertziennes dans les cellules et sur le système nerveux. Ces travaux ont été poursuivis en France, plus tard, par d'Arsonval.

En 1970, le médecin français Fellus constata que les ondes électromagnétiques, entre autres effets, augmentent la charge électrique des cellules. On sait en effet que dans les cellules vivantes saines, il existe une différence de potentiel entre le noyau et la membrane, qui est de 90 mV pour les cellules des centres nerveux et de 70 mV pour les autres cellules. Lorsque les cellules sont malades, leur réserve d'énergie est diminuée et la différence de potentiel peut tomber à 55-50 mV; si cette différence de potentiel passe au-dessous de 30 mV, la cellule meurt.

Lorsque les cellules se "déchargent", à la façon d une batterie électrique, une douleur est ressentie dans l'organisme (douleur dans les os, dans les articulations, dans le dos, etc..).

En soumettant à des ondes électromagnétiques des cellules ayant subi une telle décharge, on peut ramener à la normale leur différence de potentiel, comme l'avait découvert Fellus. Selon le cas et le type de cellules, le traitement peut demander de quelques jours à plusieurs semaines. Le résultat de ce retour à la normale de la différence de potentiel cellulaire est de faire disparaître l'inflammation douloureuse d'une articulation ou due à une distorsion; un rhumatisme peut disparaître après quelques séances, tandis que la guérison d'une fracture peut nécessiter des semaines.

L'action des ondes électromagnétiques est aussi bénéfique dans d'autres cas (infections dermatologiques, arthrites, réduction du taux de cholestérol dans le sang, normalisation de la pression sanguine, action sédative dans le système nerveux, élimination des rides et de la cellulite par exemple).

L'électromagnétothérapie est applicable à tous les mammifères supérieurs.

Les docteurs Kraus et Lechner ont été les premiers, en 1972, à adopter cette thérapie, avec un grand succès. Ainsi s'est trouvé confirmé le fait que les ondes électromagnétiques sont capables de "repolariser" les cellules.

Les appareils dits à ondes courtes (diathermie) actuellement utilisés ont l'inconvénient d'exiger des puissances élevées (de l'ordre du KW), ce qui est dangereux et exige la surveillance d'un spécialiste. De plus, ces appareils ne conviennent que pour le traitement de certaines cellules, donc de certains cas de douleurs seulement.

Le document FR-A- 2 291 773 se réfère à un appareil émetteur de signaux d'une haute fréquence rythmé et réglable tel que défini dans le préambule de la revendication 1.

Les signaux sont émis par trains d'onde pendant des durées de 10 à 500 microsecondes. Cette émission rythmée par train d'onde a lieu pendant une seconde et est inhibée pendant la seconde suivante et ainsi de suite. Le rayonnement émis est d'une puissance élevée réglable entre 10 watts et 5 kilowatts. La fréquence de ce rayonnement est réglable, mais le rayonnement émis est constitué d'une seule fréquence.

Le document BE-A- 677 767 concerne un appareil d'électrothérapie émettant des décharges discontinues d'impulsions modulées en haute fréquence à partir d'un tampon avec une puissance élevée d'environ 10 watts. Le signal électrique émis est constitué par un courant à fréquence radio créé par une alimentation de puissance qui excite un oscillateur produisant des fréquences fondamentales, leurs harmoniques et des fréquences intermodulées de valeurs discrètes entre 200 kHz et 1 gigahertz.

Ce signal se manifeste sous forme de courants à haute fréquence passant à l'intérieur des structures des tissus et des cellules, ainsi que de leurs constituants.

Signalons encore le document FR-A- 2 386 912 qui décrit un appareil d'émission d'ondes électromagnétiques comportant une antenne sous forme d'une spirale conductrice imprimée sur une feuille isolante et reliée a un générateur de signaux de haute fréquence.

La présente invention vise à fournir un appareil d'électromagnétothérapie capable de ramener à la normale la différence de potentiel cellulaire de tous les types de cellules de l'organisme et qui travaille à très faible puissance, donc exempt de danger et qui puisse être portatif et fonctionner avec des batteries rechargeables.

L'appareil selon l'invention est caractérisé à cet effet par le fait que le générateur est agencé de façon que le dispositif émetteur est alimenté avec une puissance de l'ordre du watt, par le fait qu'il comprend des troisièmes moyens pour moduler ladite onde mégahertzienne agencés de façon à produire un spectre de fréquences de modulation couvrant de façon sensiblement continue une bande allant de la fréquence de l'onde mégahertzienne a une fréquence au moins égale à deux gigaghertz, de façon que le spectre de fréquence de l'onde mégahertzienne modulée résultant comprenne des fréquences de Fourier convenant au traitement de tous les types de cellules de mammifères, par le fait que lesdits premiers moyens sont agencés de façon à émettre des trains de l'onde mégahertzienne modulée d'une première durée de l'ordre des microsecondes intercalés entre des intervalles d'interruption d'une seconde durée de l'ordre des microsecondes et par le fait que ladite troisième durée prédéterminée des pauses est de l'ordre des millisecondes.

Le dessin annexé représente, à titre d'exemple, une forme d'exécution de l'appareil selon l'invention.

Fig. 1 est une vue en perspective du boîtier de l'appareil.

Fig. 2 est un schéma bloc du circuit électronique contenu dans ce boîtier.

Fig. 3 est un schéma électrique.

Fig. 4 représente des trains d'ondes.

Fig. 5 représente à plus grande échelle, un exemple d'onde de ces trains.

Fig. 6 représente un spectre des fréquences présentes dans l'onde modulée selon fig. 5.

Fig. 7 représente schématiquement deux groupes successifs de 32 trains d'ondes selon fig. 4, séparés par une période de pause intermédiaire, chaque train étant représenté par un trait vertical.

Le boitier 1 comporte une série de six LED 2 servant à indiquer le programme en cours. Ce programme est sélectionné par un bouton 3. Un bouton 4 sert à la mise en fonction de l'appareil et d'une sortie 6a. Un autre bouton, 5, sert à la mise en fonction d'une sortie 6b.

Les sorties 6a et 6b servent à alimenter deux électrodes permettant de traiter simultanément deux parties du corps.

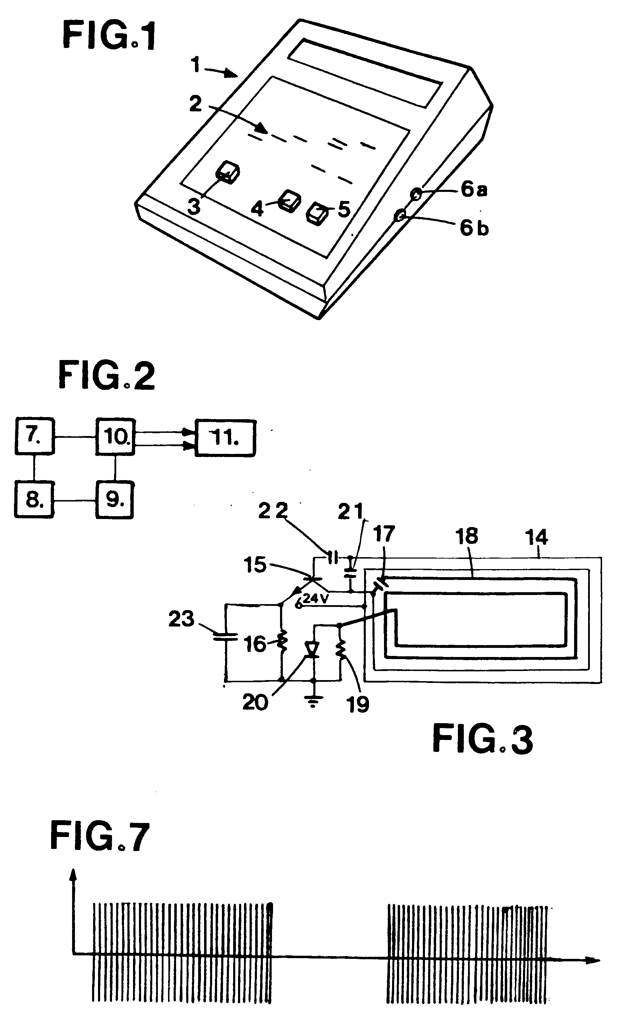

Sur la fig. 2, on a représenté en 7 une alimentation (comprenant soit le système classique transformateur et pont redresseur, soit accumulateurs avec chargeur automatique). 8 est un timer réalisé avec un circuit intégré en technologie LOCMos, avec résistance et condensateur, qui détermine et règle le temps de fonctionnement des quatre programmes suivants :
a = Haut; b = haut/moyen, c = moyen/bas; d = bas.

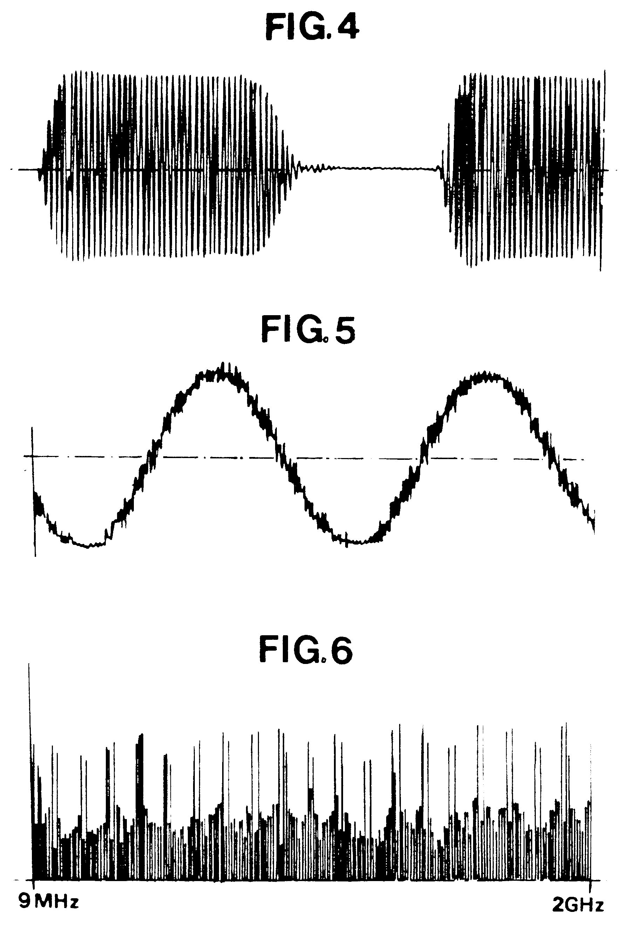

Le programme a correspond à des petits intervalles de repos entre les groupes de trains d'ondes selon fig. 7. Dans cet exemple il y a 32 trains selon fig. 4 dans chaque groupe de trains. Le programme d correspond à des grands intervalles de repos entre groupes de trains, et les programmes b et c correspondent à des intervalles de repos compris entre les deux précédents (a et d), les intervalles b étant plus courts que les intervalles c.

A la fin de chaque programme une impulsion électrique est envoyée à un commutateur 9, pour arrêter le fonctionnement de l'appareil, dans le cas des programmes a et d, ou pour passer au programme suivant dans le cas des programmes b et c, et pour ensuite arrêter le fonctionnement de l'appareil. 10 est un générateur de fréquences à circuit oscillant (inductance et capacité) à 140 KHz. Cette fréquence est divisée par un diviseur non représenté (circuit intégré LOCMos) en différentes valeurs, pour obtenir, avec un circuit flip-flop, des ensembles de 32 trains d'impulsions comme on le verra plus loin. 11 est le générateur de champ électromagnétique.

Les sorties 6a, 6b du boîtier 1 (fig. 1) servent chacune à relier au moyen d'un câble, un générateur 11 de champ électromagnétique au boîtier 1.

Sur la fig. 3, on voit un transistor 15 de haute fréquence qui oscille librement et qui génère la haute fréquence de 9 MHz. Ce transistor est mis en et hors fonction par deux autres transistors en cascade (non représentés) qui fonctionnent comme interrupteur à la fréquence de 140 KHz. Le transistor 15 est connecté, avec sa base et son collecteur à une inductance 14 en forme de spirale, en circuit imprimé porté par un support flexible du type Kapton et qui fonctionne comme inductance oscillatrice. Elle est reliée, d'une part, à une alimentation (24 V) et d'autre part (par l'intermédiaire d'une capacité 17 d'accouplement, de contrôle et de transfert d'énergie de 82 pf) à une autre inductance 18 en forme de spirale concentrique à la première et réalisée aussi en circuit imprimé. Cette inductance 18 est reliée à la masse par une résistance limitatrice 19 à charbon, de 51 ohm et une diode LED 20 de 10 mA en parallèle avec elle, et qui indique si le circuit fonctionne. Cette deuxième inductance, 18, (représentée en traits plus épais sur la fig. 3) sert, avec la première qui agit sur elle comme le primaire d'un auto-transformateur, à créer le champ électromagnétique utilisé pour les traitements. L'émetteur du transistor 15 est relié à la masse par l'intermédiaire d'une résistance limitatrice 16 de 50 ohm, en parallèle avec laquelle se trouve une capacité 23 de 10 nf.

Sur la fig. 3 on voit encore deux condensateurs 21 (de 56 pf) et 22 (de 100 pf). La disposition selon fig. 3 a pour effet de survolter considérablement la diode 20 qui, alors, sous l'effet de ce survoltage, génère une multitude de fréquences - comme il est connu - qui modulent la fréquence fondamentale de 9 MHz (fig. 5). L'expérience a montré que le spectre de fréquences du courant ainsi modulé s'étend de la fréquence fondamentale (porteuse) de 9 MHz à des fréquences de plusieurs GHz.

C'est ce courant modulé qui parcourt les inductances 14 et 18, qui, de ce fait, émettent un champ électromagnétique synchrone, donc présentant un tel spectre très étendu de fréquences (fig. 6).

Sur la fig. 4 on a montré deux trains d'ondes successifs d'une durée de 3 »s chacun et séparés par une pause intermédiaire de 2 »s. Il est en effet indispensable pour réaliser un traitement satisfaisant, que les trains d'ondes se suivent avec des périodes de repos entre eux.

Sur la fig. 5, on a représenté, à beaucoup plus grande échelle du temps, la courbe de l'onde, c'est à dire la porteuse et d'une quantité d'ondes de fréquences plus élevées (dues à la présence de la diode survoltée 20) , qui modulent cette porteuse. Cette courbe est obtenue par un oscillographe et correspond au champ émis par les inductances 14 et 18.

Sur la fig. 6, on voit le spectre des fréquences de Fourier présentes dans l'onde modulée selon fig. 4. L'échelle s'étend de la valeur de la porteuse (9 MHz) à 2 GHz. En fait, elle peut s'étendre au-delà, par exemple jusqu'à 5GHz. On voit que le spectre des fréquences donné par l'analyse de Fourier couvre pratiquement une bande s'étendant de la fréquence fondamentale aux plus hautes fréquences (de plusieurs GHz) convenant pour la repolarisation des différents types de cellules des mammifères.

Ainsi l'appareil fournit à l'organisme traité un ensemble quasi continu de fréquences parmi lesquelles se trouvent toutes celles nécessaires pour repolariser tous les types de cellules.

Le commutateur 9 est un circuit intégré qui commande quatre interrupteurs et sert à commuter les quatre durées a, b, c, d, d'intervalles entre les groupes de trains d'impulsions. Ces quatre différents intervalles ou pauses sont nécessaires selon le traitement. Le temps de repolarisation varie selon l'état des cellules et le genre de douleur à traiter. Ainsi par exemple, pour une sciatique on utilisera le programme "bas" et pour une tendinite le programme "haut". Les intervalles courts peuvent être de 1,6 ms et les intervalles longs de 13 ms, par exemple.

L'appareil décrit fonctionne à très basse puissance, de l'ordre du watt. Avec l'alimentation à 24 V, l'intensité dans les inductances 14 et 18 est de l'ordre des mA. L'appareil est donc sans danger et peut être porté facilement.

Les électrodes dans lesquelles sont incorporées les inductances rayonnantes 14 et 18 étant souples, elles peuvent être appliquées directement sur les parties à traiter, en prenant la forme de ces parties. La grandeur de ces électrodes sera choisie en fonction des parties à traiter. On disposera donc d'électrodes de grandeurs différentes, capables de produire des champs d'intensités différentes.

## Revendications

1. Appareil d'électromagnétothérapie, comprenant un générateur d'ondes électromagnétiques (fig. 3) susceptible de générer une onde hertzienne, des premiers moyens (10) pour produire l'émission de trains de cette onde hertzienne d'une première durée prédéterminée intercalés entre des intervalles d'interruption d'une seconde durée prédéterminée et des seconds moyens (9) pour inhiber l'émission desdits trains pour former des pauses d'une troisième durée prédéterminée réglable, le générateur comportant au moins un dispositif émetteur (14, 18), caractérisé par le fait que le générateur est agencé de façon que le dispositif émetteur est alimenté avec une puissance de l'ordre du watt, par le fait qu'il comprend des troisièmes moyens (20) pour moduler ladite onde hertzienne agencés de façon à produire un spectre de fréquences de modulation couvrant de façon sensiblement continue une bande allant de la fréquence de l'onde hertzienne à une fréquence au moins égale à deux gigahertz, de façon que le spectre de fréquence de l'onde hertzienne modulée résultant comprenne des fréquences de Fourier convenant au traitement de tous les types de cellules de mammifères, par le fait que lesdits premiers moyens (10) sont agencés de façon à émettre des trains de l'onde hertzienne modulée d'une première durée de l'ordre des microsecondes intercalées entre des intervalles d'interruption d'une seconde durée de l'ordre des microsecondes et par le fait que ladite troisième durée prédéterminée des pauses est de l'ordre des millisecondes.

2. Appareil selon la revendication 1, caractérisé par le fait que les troisièmes moyens (20) comportent une diode LED fortement survoltée.

3. Appareil selon la revendication 1, caractérisé par le fait que le dispositif émetteur comprend deux enroulements (17, 18) en spirales concentriques.

4. Appareil selon la revendication 3, caractérisé par le fait que les deux enroulements (17, 18) sont connectés l'un à l'autre par une capacité d'accouplement (17).

## Claims

1. An electromagnetotherapeutic apparatus, including a generator of electromagnetic waves (fig. 3) capable of generating a hertzian wave, first means (10) for ensuring the emission of trains of this hertzian wave of a first predetermined duration spaced by intervals of interruption of a second predetermined duration and second means (9) for inhibiting the emission of said trains to form pauses of a third predetermined adjustable duration, the generator including at least one emitter device (14, 18), characterized by the fact that the generator is arranged in such a manner that the emitter device is supplied with a power in the order of the watt, in that it includes third means (20) for modulating said hertzian wave, arranged in such a manner as to produce a spectrum of modulation frequencies covering substantially continuously a band extending from a frequency of the hertzian wave to a frequency of at least two gigahertz, so that the spectrum of frequency of the resulting modulated hertzian wave includes Fourier frequencies adapted to the treatment of all types of mammalian cells, in that said first means (10) are arranged in such a manner as to emit trains of modulated hertzian waves of a first duration of the order of microseconds spaced by intervals of interruption of a second duration in the order of the microseconds and in that said third predetermined duration of the pauses is in the order of milliseconds.

2. An apparatus according to claim 1, characterized by the fact that the third means (20) include a LED operating under a high overvoltage.

3. An apparatus according to claim 1, characterized by the fact that the emitter device includes two windings (17, 18) arranged in concentric spires.

4. An apparatus according to claim 3, characterized in that the two windings (17, 18) are connected together by a coupling capacitor (17).

## Patentansprüche

1. Gerät zur elektromagnetischen Therapie mit einem zur Erzeugung einer Hertzschen Welle geeigneten elektromagnetischen Wellengenerator (Figur 3), ersten Mitteln (10), um die Aussendung von Wellenzügen dieser Hertzschen Welle zu bewirken, die eine erste vorbestimmte Dauer haben und in Unterbrechungszeitintervalle einer zweiten, vorbestimmten Dauer fallen, sowie zweiten Mitteln (9), um die Aussendung der benannten Wellenzüge zu unterbinden, um Pausen einer dritten, einstellbaren vorbestimmten Dauer zu bilden, wobei der Generator zumindest eine Sendevorrichtung (14, 18) umfasst, dadurch gekennzeichnet, dass der Generator so eingerichtet ist, dass die Sendevorrichtung mit einer Leistung von der Grössenordnung von Watt gespeist wird; dadurch, dass es dritte Mittel (20) umfasst, um die benannte. Hertzsche Welle zu modulieren, wobei diese Mittel so eingerichtet sind, dass sie ein Modulationsfrequenzspektrum hervorbringen, das in im wesentlichen kontinuierlicher Weise ein Band überstreicht, das von der Frequenz der Hertzschen Welle bis zu einer Frequenz von zumindest zwei Gigahertz reicht, so dass das Frequenzspektrum der sich ergebenden, modulierten Hertzschen Welle Fourierfrequenzen umfasst, die sich zur Behandlung aller Typen von Säugetierzellen eignen; dadurch, dass die benannten ersten Mittel (10) so eingerichtet sind, dass sie Wellenzüge der modulierten Hertzschen Welle von einer ersten Dauer in der Grössenordnung von Mikrosekunden aussenden, die in Unterbrechungszeitintervalle einer zweiten Dauer in der Grössenordnung von Mikrosekunden fallen; und dadurch, dass die benannte vorbestimmte dritte Dauer von Pausen von der Grössenordnung von Millisekunden ist.

2. Gerät gemäss Anspruch 1, dadurch gekennzeichnet, dass die dritten Mittel (20) eine stark hinauftransformierte Diode LED umfassen.

3. Gerät gemäss Anspruch 1, dadurch gekennzeichnet, dass die Sendevorrichtung zwei konzentrisch gewundene Wicklungen (17, 18) aufweist.

4. Gerät gemäss Anspruch 3, dadurch gekennzeichnet, dass die beiden Wicklungen (17, 18) miteinander durch eine Kopplungskapazität (17) verbunden sind.
